# EUROPÄISCHE PATENTSCHRIFT

(11) **EP 0 605 493 B1**
(45) Veröffentlichungstag und Bekanntmachung des Hinweises auf die Patenterteilung: **05.12.2001**
(21) Anmeldenummer: 92919432.2
(22) Anmeldetag: 17.09.1992
(51) Int. Cl.: C07D 513/04, A61K 31/435

(54) **ANTIALLERGISCHE THIADIAZOLO(4,3-a)PYRIDINDERIVATIVE**
THIADIAZOLO (4,3-a)PYRIDINE DERIVATIVES, METHODS OF PREPARING THEM AND DRUGS CONTAINING THEM
DERIVES DE LA THIADIAZOLO( 4,3-a)PYRIDINE, PROCEDES DE PREPARATION ET MEDICAMENTS CONTENANT CES DERIVES

(30) Priorität: 23.09.1991 DE 4131579
(43) Veröffentlichungstag der Anmeldung: 13.07.1994
(73) Patentinhaber: Roche Diagnostics GmbH, 68298 Mannheim (DE)
(72) Erfinder: FRIEBE, Walter-Gunar, D-6800 Mannheim 1 (DE); WILHELMS, Henning, D-6941 Weinheim/Rittenweier (DE)
(74) Vertreter: Weber, Manfred, Dr.
(86) Internationale Anmeldenummer: EP9202142
(87) Internationale Veröffentlichungsnummer: WO9306109

(56) Entgegenhaltungen:
- GB-A- 2 163 427
- US-A- 4 775 677
- JOURNAL OF ORGANIC CHEMISTRY. Bd. 36, Nr. 13, 1971, EASTON US Seiten 1846 - 1848 K. T. POTTS ET AL. 'Bridgehead nitrogen heterocycles. V. Some 3H-(1,2,4)thiadiazolo(4,3-a)pyridines derived from 2-trichloromethylthioaminopyridine' in der Anmeldung erw{hnt
- JOURNAL OF ORGANIC CHEMISTRY. Bd. 35, Nr. 6, 1970, EASTON US Seiten 1965 - 1968 K. T. POTTS ET AL. 'Bridgehead nitrogen heterocycles. III. The 3H (1,2,4)thiadiazolo(4,3-a)pyridine system' in der Anmeldung erw{hnt

## Beschreibung

Gegenstand der vorliegenden Erfindung sind Thiadiazolo[4,3-a]pyridinderivate, Verfahren zu deren Herstellung und Arzneimittel, die diese Verbindungen enthalten.

Die Erfindung betrifft Thiadiazolo[4,3-a]pyridinderivate der allgemeinen Formel I 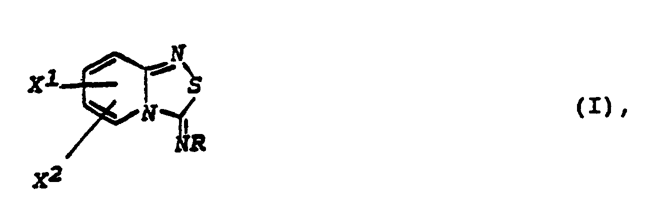 in welcher
- X¹ und X²: gleich oder verschieden Wasserstoff, einen C₁- bis C₆-Alkylrest oder ein Halogenatom bedeuten oder, sofern sie in benachbarten Positionen stehen, gemeinsam mit den sie tragenden Kohlenstoffatomen einen ankondensierten Phenylring bilden, und
- R: einen Phenyl-, Cyclohexyl-, Cyclopentyl-, Pyridyl-, Piperidinyl-, Pyrimidinyl-, Pyrrolidinyl-, Isoxazolyl-, Oxazolyl-, Thiazolyl, Thiazolinyl-, Triazolyl- oder Tetrazolylrrest, der gewünschtenfalls ein- oder mehrfach durch Halogen, Cyano, Nitro, C₁- bis C₆-Alkyl, C₁- bis C₆-Halogenalkyl, Hydroxy, C₁- bis C₆-Alkoxy, Methylendioxy, C₁- bis C₆-Alkylthio, C₁- bis C₆-Halogenalkylthio, Amino, C₁- bis C₆-Alkylamino, C₂- bis C₁₂-Dialkylamino, Pyrrolyl, Carboxy, Carbamoyl, Benzyl, C₁- bis C₆-Hydroxyalkyl, C₂- bis C₇-Carboxyalkyl, C₂-bis C7-Alkoxycarbonyl-C₁- bis C₆-alkyl, Carbamoyl-C₁-bis C₆-alkyl, N-Hydroxy-N-C₁- bis C₆-alkyl-carbamoyl-C₁-bis C₆-alkyl oder C₂- bis C₆-Alkenyl substituiert sein kann bedeutet sowie deren physiologisch verträgliche Salze,
mit der Maßgabe, daß R nicht 2-Pyridinyl bedeuten kann und nicht Phenyl, 2,5-Dichlorphenyl oder 4-Methylphenyl bedeuten kann, wenn X¹ und X² gleichzeitig Wasserstoff sind, oder R nicht 3,4-Dichlorphenyl bedeuten kann, wenn X¹ oder X² Methyl darstellt.

Es wurde überraschend gefunden, daß die Verbindungen der Formel I wertvolle pharmakologische Eigenschaften aufweisen. Insbesondere können sie die antigenbedingte Kontraktion von Lungengewebestreifen hemmen. Sie eignen sich daher zur Behandlung allergischer Krankheiten sowie entzündungsbedingten bronchospastischen und bronchokonstriktorischen Reaktionen.

Außerdem können sie die Letalität eines Endotoxin-Schocks verhindern und eignen sich daher zur Behandlung von durch Monokine hervorgerufenen entzündlichen Prozessen sowie septischer Schock, Autoimmunerkrankungen, graft-versus-host-Reaktionen und host-versus-graft-Erkrankungen.

In J. Org. Chem. 35, 1965 (1970) ist u. a. die Verbindung 3-(2- Pyridinylimino)-3H-[1,2,4]-thiadiazolo [4,3-a]-pyridin, in J. Org. Chem. 36, 1846 (1971) die Verbindungen 3-Phenylimino-3H-[1,2,4]-thiadiazolo [4,3-a]-pyridin und 3-(4-Methyl-phenylimino)-3H-[1,2,4]-thiadiazolo [4,3-a]-pyridin beschrieben, jedoch ohne Angabe einer pharmakologischen Wirksamkeit. Diese Verbindungen sind auch als Arzneimittel einsetzbar und Gegenstand der Erfindung.

Die Alkylreste in den genannten Alkyl-, Alkoxy-, Alkylthio- und (Di)Alkylaminogruppen sowie die Alkenylreste können geradkettig oder verzweigt sein. Bevorzugte Alkylreste in diesen Gruppen sind der Methyl- Ethyl-, n-Propyl-, Isopropyl-, n-Butyl-, Isobutyl-, tert.-Butyl-, n-Pentyl- und 3-Pentylrest, bevorzugte Alkenylreste der Vinyl- und der Allylrest.

Ein C₁-C₆ Halogenalkylrest ist bevorzugt Trifluormethyl.

Als Halogenatome kommen Fluor, Chlor und Brom in Frage.

Bevorzugte Verbindungen der Formel I sind Verbindungen, in denen X¹ Wasserstoff, Methyl oder Chlor, X² Wasserstoff oder X¹ und X² zusammen einen ankondensierten Phenylring bilden und R einen Phenylring, der ein- oder zweifach durch Fluor, Chlor, Methyl, Methoxy, tert. Butyl, Isopropoxy, Trifluormethyl, Trifluormethylthio, Hydroxy, Nitril, Nitro, Hydroxymethyl, Methylendioxy, Diethylamino, Methylthio, Pyrrolyl, Methoxycarbonylmethyl, Carboxymethyl, N-Hydroxy-N-methyl-carbamoylmethyl oder N-Tetrazolyl-carbamoylmethyl substituiert sein kann, oder einen Thiazolyl-, Pyridinyl,- Tetrazolyl,- Pyrimidyl- oder Cyclohexyl-Rest darstellt.

Außer den in den Beispielen genannten Verbindungen sind Gegenstand der Erfindung insbesondere alle Substanzen, die jede mögliche Kombination der in den Beispielen genannten substituenten aufweisen.

Das erfindungsgemäße Verfahren zur Herstellung der Verbindungen der Formel I ist dadurch gekennzeichnet, daß man in an sich bekannter Weise eine Verbindung der allgemeinen Formel II 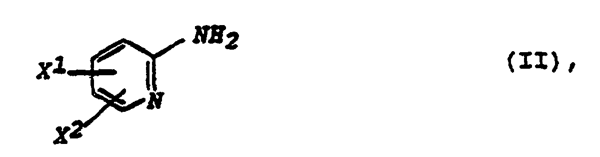 in der X¹ und X² die obengenannte Bedeutung haben, entweder
a) mit einer Verbindung der Formel III

   ClSCCl₃ (III),

   oder einem reaktiven Derivat davon
   und einer Verbindung der allgemeinen Formel IV

   H₂N-R (IV),

   in der R die obengenannte Bedeutung hat, oder
b) mit einer Verbindung der allgemeinen Formel V

   YS-CY=N-R (V),

   in der R die obengenannte Bedeutung hat und Y ein Halogenatom darstellt, umsetzt und anschließend gewünschtenfalls einen Rest R in einen anderen durch die Definition gegebenen Rest überführt und die erhaltene Verbindung der Formel I gewünschtenfalls durch Umsetzung mit physiologisch verträglichen Säuren oder Basen in ein Salz umwandelt.

Als Halogenatome für Y kommen insbesondere Chlor und Brom in Frage.

Das erfindungsgemäße Verfahren führt man vorzugsweise so durch, daß man zunächst eine Verbindung der allgemeinen Formel II mit einer Verbindung der Formel III kondensiert und das erhaltene Produkt isoliert. Dieses Zwischenprodukt wird dann mit einer Verbindung der allgemeinen Formel IV zur Reaktion gebracht.

Eine andere Variante besteht darin, das aus der Umsetzung einer Verbindung der Formel II mit einer Verbindung der Formel III erhaltene Reaktionsgemisch ohne Isolierung des Zwischenprodukts mit einer Verbindung der Formel IV reagieren zu lassen.

Die Reaktionen erfolgen zweckmäßig in einem Lösungsmittel wie Wasser, Ether, einem niederen Alkohol wie beispielsweise Methanol oder Ethanol oder einem halogenierten Kohlen-wasserstoff wie Dichlormethan oder Trichlormethan unter Zugabe einer Base wie Triethylamin oder Natriumcarbonat bei Temperaturen zwischen -20 und 50°C, vorzugsweise zwischen 0°C und Raumtemperatur.

Die Verbindungen der Formeln II, IV und V sind aus der Literatur bekannt oder können von bekannten Verbindungen ausgehend nach trivialen Methoden leicht hergestellt werden.

Eine Umwandlung eines Restes R in einen anderen Rest R erfolgt beispielsweise durch Etherspaltung mit einer Protonensäure oder Lewis-Säure wie Bromwasserstoff, Chlorwasserstoff, Iodwasserstoff, Aluminiumtrichlorid, Bortribromid oder durch Alkylierung einer Hydroxygruppe mit dem gewünschten Alkylhalogenid. oder Alkylsulfat.

Eine in R enthaltene Carboxygruppe kann, gewünschtenfalls über ein reaktives Derivat wie ein Halogenid, Imidazolid oder Anhydrid, in eine Estergruppe oder Carbonamidfunktion überführt werden; eine in R enthaltene Estergruppe läßt sich durch saure oder basische Hydrolyse in die Carboxygruppe, durch Aminolyse in die Carbonamidgruppe umwandeln.

Als pharmakologisch verträgliche Salze kommen insbesondere Alkali-, Erdalkali- und Ammoniumsalze sowie gegebenenfalls Salze mit nichttoxischen anorganischen oder organischen Säuren, wie z.B. Salzsäure, Schwefelsäure, Phosphorsäure, Bromwasserstoffsäure, Essigsäure, Milchsäure, Zitronensäure, Äpfelsäure, Benzoesäure, Salicylsäure, Malonsäure, Maleinsäure, Bernsteinsäure oder Diaminocapronsäure in Frage.

Die Salze erhält man in üblicher Weise z.B. durch Neutralisation der Verbindungen der Formel I mit den entsprechenden Laugen oder Säuren.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung; welches die bei Injektionslösungen üblichen Stabilisierungsmittel, Lösungsvermittler und / oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Geschmacks- und Süßstoffe enthalten. Für die äußerliche Anwendung können die erfindungsgemäßen Substanzen I auch in Form von Pudern und Salben verwendet werden. Sie werden dazu z.B. mit pulverförmigen, physiologisch verträglichen Verdünnungsmitteln bzw. üblichen Salbengrundlagen vermischt.

Die verabreichte Dosis hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg/Tag in einer oder mehreren Anwendungen pro Tag wirksam, um die gewünschten Resultate zu erhalten.

Außer den in den Beispielen genannten Substanzen sind im Sinne der vorliegenden Erfindung die folgenden Verbindungen bevorzugt:
1. 4-(3H-[1,2,4]-Thiadiazolo[4,3-a]pyridin-3-ylidenamino)phenylessigsäure-N-(1H-tetrazol-5-yl)amid
2. 3-(2-Pyrimidinyl-imino)-3H-[1,2,4]-thiadiazolo[4,3-a]-pyridin
3. 5-Methyl-3-(4-pyridinyl-imino)-3H-[1,2,4]-thiadiazolo-[4,3-a]pyridin
4. 6-Methyl-3-(4-pyridinyl-imino)-3H-[1,2,4]-thiadiazolo-[4,3-a]pyridin
5. 8-Methyl-3-(4-pyridinyl-imino)-3H-[1,2,4]-thiadiazolo-[4,3-a]pyridin

### Beispiel 1

### 3-(Thiazol-2-yl-imino)-3H-[1,2,4]-thiadiazolo[4,3-a]pyridin

Zu einer Lösung von 6.6 ml (60 mmol) Trichlormethansulfensäurechlorid in 900 ml Chloroform tropft man bei 0°C eine Lösung von 5.6 g (60 nmol) 2-Amino-pyridin und 8.3 ml Triethylamin in 50 ml Chloroform. Man rührt 10 min nach und tropft eine Lösung von 6.0 g (60 mmol) 2-Amino-thiazol und 25 ml Triethylamin in 100 ml Chloroform zu. Nach 3 h Rühren bei Raumtemperatur engt man ein und wäscht den Niederschlag mit Methanol. Es verbleiben 8.9 g Titelverbindung (63 % d.Th.) vom Schmp. 169-171°C.

### Beispiel 2

In analoger Weise wie in Beispiel 1 beschrieben erhält man aus Trichlormethansulfensäurechlorid, 2-Amino-pyridin und dem jeweiligen Amin:

| Bezeichnung | | Ausb. % | Schmelzpunkt °C (Lsg.mittel) |
|---|---|---|---|
| a) | 3-(2-Pyridinyl-imino)-3H-[1,2,4]-thiadiazolo[4,3-a]-pyridin aus 2-Amino-pyridin | 80 | 159-161 (Ether) |
| b) | 3-(4-Pyridinyl-imino)-3H-[1,2,4]-thiadiazolo[4,3-a]-pyridin aus 4-Amino-pyridin | 37 | 185-186 (Ethanol) |
| c) | 3-Phenylimino-3H-[1,2,4]-thiadiazolo[4,3-a]pyridin aus Anilin | 67 | 84-86 (2-Propanol) |
| d) | 3-(4-Chlor-phenylimino)-3H-[1,2,4]-thiadiazolo[4,3-a]-pyridin aus 4-Chlor-anilin | 64 | 129-130 (2-Propanol) |
| e) | 3-(4-Methoxy-phenylimino)-3H-[1,2,4]-thiadiazolo-[4,3-a]pyridin aus 4-Methoxy-anilin | 69 | 90-92 (2-Propanol) |
| f) | 3-(4-Cyano-phenylimino)-3H-[1,2,4]-thiadiazolo[4,3-a]-pyridin aus 4-Amino-benzonitril | 57 | 149-150 (2-Propanol) |
| g) | 3-(4-Nitro-phenylimino)-3H-[1,2,4]-thiadiazolo[4,3-a]pyridin aus 4-Nitro-anilin | 63 | 198-199 (2-Propanol) |
| h) | 3-Cyclohexylimino-3H-[1,2,4]-thiadiazolo[4,3-a]pyridin-hydrochlorid aus Cyclohexylamin | 28 | 194-196 (Ethylacetat) |
| i) | 3-(5-1H-Tetrazolyl-imino)-3H-[1,2,4]-thiadiazolo-[4,3-a]pyridin aus 5-Amino-1H-tetrazol | 29 | 282-283 (Wasser) |
| j) | 3-(4-Fluor-phenylamino)-3H-[1,2,4]-thiadiazolo[4,3-a]pyridin aus 4-Fluor-anilin | 67 | 138-140 (2-Propanol) |
| k) | 3-(2,4-Dichlor-phenylimino)-3H-[1,2,4]-thiadiazolo-[4,3-a]pyridin aus 2,4-Dichlor-anilin | 51 | 130-132 (2-Propanol) |
| l) | 3-(4-Methyl-phenylimino)-3H-[1,2,4]-thiadiazolo[4,3-a]pyridin aus 4-Methyl-anilin | 59 | 106-108 (2-Propanol) |
| m) | 3-(3-Trifluormethyl-phenyl-imino)-3H-[1,2,4]-thiadiazolo[4,3-a]pyridin aus 3-Trifluormethyl-anilin | 34 | 48-49 (2-Propanol)) |
| n) | 3-(2-Hydroxymethyl-phenylimino)-3H-[1,2,4]-thiadiazolo[4,3-a]pyridin aus 2-Amino-benzylalkohol | 49 | 118-119 (2-Propanol) |
| o) | 3-(2-Hydroxy-phenylimino)-3H-[1,24]-thiadiazolo[4,3-a]-pyridin aus 2-Amino-phenol | 21 | 130-132 (2-Propanol) |
| p) | 3-(4-Hydroxy-2-methyl-phenyliaino)-3H-[1,2,4]-thiadiazolo[4,3-a]pyridin aus 4-Amino-3-methyl-phenol | 49 | 241-243 (2-Propanol) |
| q) | 3-(3,4-Methylendioxy-phenylimino)-3H-[1,2,4]-thiadiazolo[4,3-a]pyridin aus 3,4-Methylendioxy-anilin | 49 | 141-143 (2-Propanol) |
| r) | 3-(3-Trifluormethylthio-phenylimino)-3H-[1,2,4]-thiadiazolo[4,3-a]pyridin aus 3-Trifluormethylthio-anilin | 70 | 86-88 (2-Propanol) |
| s) | 3-(4-Diethylamino-phenylimino)-3H-[1,2,4]-thiadiazolo[4,3-a]pyridin aus N,N-Diethyl-1,4-phenylendiamin | 54 | 96-97 (2-Propanol) |
| t) | 3-(5-Methyl-isoxazol-3-yl-imino)-3H-[1,2,4]-thiadiazolo[4,3-a]pyridin aus 3-Amino-5-methyl-isoxazol | 36 | 170-172 (2-Propanol) |
| u) | 3-(4,5-Dihydro-thiazol-2-yl-imino)-3H-[1,2,4]-thiadiazolo(4,3-a]pyridin aus 2-Amino-2-thiazolin | 55 | 106-108 (2-Propanol) |
| v) | 3-(1-Benzyl-piperidin-4-yl-imino)-3H-[1,2,4]-thiadiazolo[4,3-a]pyridin aus 4-Amino-1-benzyl-piperidin | 55 | 90-92 (2-Propanol) |
| w) | 3-(3-Pyridinyl-imino)-3H-[1, 2,4]-thiadiazolo[4,3-a]pyridin aus 3-Amino-pyridin | 67 | 133-134 (2-Propanol) |
| x) | 3-(4-t-Butyl-phenylimino)-3H-[1,2,4]-thiadiazolo-[4,3-a]pyridin aus 4-t-Butyl-anilin | 46 | 72-74 (Isohexan) |
| y) | 3-(4-Isopropoxy-phenylimino)-3H-[1,2,4]-thiadiazolo-[4,3-a]pyridin aus 4-Isopropoxy-anilin | 52 | 94-95 (Ether) |
| z) | 3-(4-Methylthio-phenylimino)-3H-[1,2,4]-thiadiazolo-[4,3-a]pyridin aus 4-Methylmercapto-anilin | 76 | 111-112 (2-Propanol) |
| aa) | 3-(4-Pyrrolidino-phenylimino)-3H-[1,2,4]-thiadiazolo[4,3 a]pyridin aus N-(4-Amino-phenyl)pyrrol | 68 | 167-168 (Ethylacetat) |

### Beispiel 3

### 7-Methyl-3-(4-pyridinyl-imino)-3H-[1,2,4]-thiadiazolo-[4,3-a]pyridin

Zu einer Lösung von 3.3 ml (30 mmol) Trichlormethansulfensäurechlorid in 450 ml Dichlormethan tropft man bei 0°C eine Lösung von 3.2 g (30 mmol) 2-Amino-4-methyl-pyridin und 4.2 ml Triethylamin 25 ml Dichlormethan, rührt 10 min und tropft eine Lösung von 2.8 g (30 mmol) 4-Aminopyridin und 12 ml Triethylamin in 100 ml Dichlormethan zu. Nach 3 h Rühren bei Raumtemperatur gießt man in Wasser, extrahiert mit Ethylacetat, trocknet den Extrakt, engt ein und chromatographiert an Kieselgel (Elutionsmittel Ethylacetat/Isohexan 1:1). Man isoliert 1.2 g Titelverbindung (17 % d.Th.) vom Schmp. 144-145°C.

### Beispiel 4

### 6-Chlor-3-(4-pyridinyl-imino)-3H-[1,2,4]-thiadiazolo-[4,3-a]pyridin

In analoger Weise wie in Beispiel 3 beschrieben erhält man die Titelverbindung mit 20 % Ausbeute vom Schmp. 205-207°C aus 2-Amino-5-chlor-pyridin und 4-Amino-pyridin.

### Beispiel 5

### 3-Phenylimino-3H-[1,2,4]-thiadiazolo[4,3-a]chinolin

Zu einer Lösung von 3.1 g (21 mmol) 2-Amino-chinolin und 6 ml Triethylamin in 70 ml Dichlormethan tropft man bei 0°C eine Lösung von 4.1 g (21 mmol) 1-Chlor-1-phenyliminomethansulfensäurechlorid in 20 ml Dichlormethan, rührt 3 h bei Raumtemperatur, wäscht die organische Phase mit Wasser, trocknet, engt ein und chromatographiert an Kieselgel. Mit Isohexan/Ethylacetat 9:1 eluiert man 2.3 g Titelverbindung (39 % d.Th.) vom Schmp. 116-118°C.

### Beispiel 6

### 3-(4-Pyridinyl-imino)-3H-[1,2,4]-thiadiazolo[4,3-a]chinolin

In analoger Weise wie in Beispiel 3 beschrieben erhält man die Titelverbindung mit 11 % Ausbeute vom Schmp. 180-182°C aus 2-Amino-chinolin und 4-Amino-pyridin.

### Beispiel 7

### 3-(4-Methoxycarbonylmethyl-phenylimino)-3H-[1,2,4]-thiadiazolo[4,3-a]pyridin

In analoger Weise wie in Beispiel 1 beschrieben erhält man die Titelverbindung mit 41 % Ausbeute vom Schmp. 151-152°C (aus Ethylacetat) aus 2-Amino-pyridin und 4-Amino-phenylessigsäuremethylester.

### Beispiel 8

### 4-(3H-[1,2,4]-Thiadiazolo[4,3-a]pyridin-3-ylidenamino)phenylessiqsäure-hydrochlorid

Eine Mischung aus 9.0 g (30 mmol) der Verbindung des Beispiels 7, 100 ml Ethanol und 300 ml N Salzsäure wird 6 h bei 50°C gerührt. Man engt ein und verreibt den Rückstand mit Aceton. Es verbleiben 8.9 g Titelverbindung (92 % d.Th.) vom Schmp. 209-211°C.

### Beispiel 9

### 4-(3H-[1,2,4]-Thiadiazolo[4,3-a]pyridin-3-ylidenamino)phenylessigsäure-N-methyl-hydroxamid

Zu einer Mischung aus 4.8 g (15 mmol) Verbindung des Beispiels 8, 90 ml Dichlormethan und 1 ml Dimethylformamid tropft man bei 0°C eine Lösung von 1.7 ml Oxalylchlorid in 15 ml Dichlormethan. Die erhaltene Lösung wird 40 min gerührt und anschließend zu einer Lösung von 5.0 g N-Methyl-hydroxylamin in 15 ml Triethylamin, 10 ml Wasser und 60 ml Tetrahydrofuran getropft. Nach 30 min Rühren versetzt man mit 20 ml Wasser, extrahiert mit Dichlormethan, trocknet, engt ein und reinigt an Kieselgel (Elutionsmittel Trichlorethan/Methanol 95:5). Man isoliert 3.4 g Titelverbindung (78 % d.Th.), die nach Verreiben mit Ether bei 135-136°C schmelzen.

### Prüfbericht

### Hemmung der Antigen-bedingten Konstriktion von passiv sensibilisierten Meerschweinchen - Lungenparenchym - Streifen in vitro (Organbad)

Für die in vitro Untersuchung der erfindungsgemäßen Verbindungen wurde die Hemmung der Antigen-bedingten Konstriktion von passiv sensibilisiertem Meerschweinchen-Lungenparenchym-Streifen gemessen, wie nachfolgend beschrieben:

Pirbright-White Meerschweinchen wurden betäubt durch Genickschlag und entblutet. Die Lungen wurden in situ mit Krebs-Puffer, pH 7.4, weitgehend blutfrei gespült. Anschließend wurde die Lunge entnommen, in Streifen geschnitten (ca. 20 x 4 x 4 mm), und die Streifen für eine Stunde bei Raumtemperatur mit einer 1:50-Verdünnung eines homologen anti-Ov-Albumin-Antiserums passiv sensibilisiert und dann mit Krebs-Puffer 1 x gewaschen. Das Antiserum war vorher nach DAVIES (1) in Meerschweinchen des gleichen Stammes erzeugt worden durch wiederholte Injektion von Ov-Albumin (2 x kristallisiert) unter Zusatz von komplettem Freund'schen Adjuvans. Bis zu seiner Verwendung wurde das Antiserum bei - 18 °C unverdünnt gelagert. Anschließend wurden die Lungenstreifen einzeln in 10 ml-Wasserbädern mit einer Vorspannung von 1.2 g an einem isometrischen Meßaufnehmer aufgehängt. Danach wurden die Bäder mit Krebs-Puffer gefüllt und kontinuierlich bei 37 °C mit O₂ (95 %) und CO₂ (5 %) begast. Über einen Verstärker wurden die Konstriktionen der Lungenstreifen auf einem Schreiber aufgezeichnet. Nach 30-minütiger Eingewöhnungsphase wurden Histamin-Kontrollspasmen zur Erkennung der Reaktionsfähigkeit der Organstücke erzeugt, gewaschen, anschließend für 20 Minuten die Prüfsubstanz bei 37 °C vorinkubiert und danach die Ov-Albumin-bedingte Konstriktion ausgelöst. Die Hemmwirkungen der erfindungsgemäßen Verbindungen wurden als prozentuale Verringerung der Konstriktionsamplitude der "Proben mit Prüfsubstanz" im Verhältnis zu den "ungehandelten Kontrollkonstriktionen" ausgedrückt.

### (1) DAVIES, G.E., T.P. Johnstone

Quantitative studies on anaphylaxis in guinea pigs passively sensitized with homologous antibody. Inter. Arch. Allergy 41, 648 - 454 (1971)

**Tabelle**

| % Hemmung der durch Ovalbumin (0,1 µg/ml) induzierten Konstriktion von passiv-sensibilisiertem Lungenparenchymstreifen (Meerschweinchen) | | |
|---|---|---|
| 20 min/37 °C Vorinkubationszeit (Organbadtechnik) | | |
| n = Anzahl der Tests | | |

| Substanz Bsp.No. | Konzentration (10 µg/ml) | n |
|---|---|---|
| Aminophyllin | 26* | 6 |
| 2a) | 56 | 3 |
| 2b) | 80 | 2 |
| 2e) | 48 | 3 |
| 2p) | 62 | 3 |
| 2q) | 57 | 3 |
| 2u) | 78 | 4 |
| 4 | 61 | 3 |
| 9 | 61 | 3 |

| | | |
|---|---|---|
| * = 200 µg/ml | | |

## Patentansprüche

1. Verbindungen der Formel 1 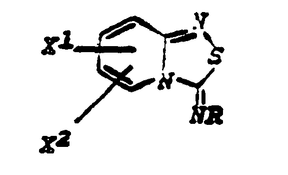 in welcher
X¹ und X² gleich oder verschieden Wasserstoff, einen C₁- bis C₆-Alkylrest oder ein Halogenatom bedeuten oder, sofern sie in benachbarten Positionen stehen, gemeinsam mit den sie tragenden Kohlenstoffatomen einen ankondensierten Phenylring bilden, und
R einen Phenyl-, Cyclohexyl-, Cyclopentyl-, Pyridyl-, Piperidinyl-, Pyrimidinyl-, Pyrrolidinyl-, Isoxazolyl-, Oxazolyl-, Thiazolyl-, Thiazolinyl-, Triazolyl- oder Tetrazolylrest, der gewünschtenfalls ein- oder mehrfach durch Halogen, Cyano, Nitro, C₁- bis C₆-Alkyl,C₁- bis C₆-Halogenalkyl, Hydroxy, C₁- bis C₆-Alkoxy, Methylendioxy, C₁- bis C₆-Alkylthio, C₁- bis C₆-Halogenalkylthio, Amino, C₁- bis C₆-Alkylamino, C₂-bis C₁₂-Dialkylamino, Pyrrolyl, Carboxy, Carbamoyl, Benzyl, C₁- bis C₆-Hydroxyalkyl, C₂- bis C₇-Carboxyalkyl, C₂- bis C₇-Alkoxycarbonyl-C₁- bis C₆-alkyl, Carbamoyl-C₁- bis C₆-alkyl, N-Hydroxy-N-C₁-bis C₆-alkyl-carbamoyl-C₁- bis C₆-alkyl oder C₂- bis C₆-Alkenyl substituiert sein kann bedeutet sowie deren physiologisch verträgliche Salze,
mit der Maßgabe, daß R nicht 2-Pyridinyl bedeuten kann und nicht Phenyl, 2,5-Dichlorphenyl oder 4-Methylphenyl bedeuten kann, wenn X¹ und X² gleichzeitig Wasserstoff sind, oder R nicht 3,4-Dichlorphenyl bedeuten kann, wenn X¹ oder X² Methyl darstellt.

2. 3-(4-Pyridinyl-imino)-3H-[1, 2, 4]-thiadiazolo [4,3-a]-pyridin gemäß Anspruch 1 sowie deren physiologisch verträglichen Salze.

3. Verfahren zur Herstellung von Verbindungen gemäß Anspruch 1, **dadurch gekennzeichnet, daß** man in an sich bekannter Weise eine Verbindung der Formel II 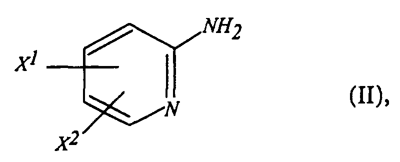 in der X¹ und X² die obengenannte Bedeutung haben, entweder
a) mit einer Verbindung der Formel III
ClSCCl₃ (III),
oder einem reaktiven Derivat davon
und einer Verbindung der allgemeinen Formel IV
H₂N-R (IV),
in der R die obengenannte Bedeutung hat, oder
b) mit einer Verbindung der allgemeinen Formel V
YS-CY=N-R (V),
in der R die obengenannte Bedeutung hat und Y ein Halogenatom darstellt, umsetzt
und anschließend gewünschtenfalls einen Rest R in einen anderen durch die Definition gegebenen Rest überführt und die erhaltene Verbindung der Formel I gewünschtenfalls durch Umsetzung mit physiologisch verträglichen Säuren oder Basen in ein Salz umwandelt.

4. Arzneimittel, enthaltend eine Verbindung der allgemeinen Formel I gemäß Anspruch 1 neben üblichen Träger- und Hilfsstoffen.

5. Arzneimittel, enthaltend die Verbindung des Anspruches 2 neben üblichen Träger- und Hilfsstoffen.

6. Verbindungen der allgemeinen Formel I gemäß Anspruch 1 zur Herstellung von Arzneimitteln zur Behandlung allergischer Krankheiten.

7. Verbindung des Anspruches 2 zur Herstellung von Arzneimitteln zur Behandlung allergischer Krankheiten.

## Claims

1. Compounds of formula 1 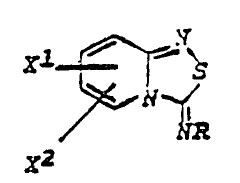 in which
X¹ and X² are the same or different and denote hydrogen, a C₁ to C₆ alkyl residue or halogen atom or, provided they are in neighbouring positions, form a condensed phenyl ring together with the carbon atoms which carry them and
R denotes a phenyl, cyclohexyl, cyclopentyl, pyridyl, piperidinyl, pyrimidinyl, pyrrolidinyl, isoxazolyl, oxazolyl, thiazolyl, thiazolinyl, triazolyl or tetrazolyl residue which can optionally be substituted once or several times by halogen, cyano, nitro, C₁ to C₆ alkyl, C₁ to C₆ halogenalkyl, hydroxy, C₁ to C₆ alkoxy, methylene dioxy, C₁ to C₆ alkylthio, C₁ to C₆ halogenalkylthio, amino, C₁ to C₆ alkylamino, C₂ to C₁₂ dialkylamino, pyrrolyl, carboxy, carbamoyl, benzyl, C₁ to C₆ hydroxyalkyl, C₂ to C₇ carboxyalkyl, C₂ to C₇ alkoxycarbonyl-C₁ to C₆ alkyl, carbamoyl-C₁ to C₆ alkyl, N-hydroxy-N-C₁ to C₆ alkyl-carbamoyl-C₁ to C₆ alkyl or C₂ to C₆ alkenyl and physiologically tolerated salts thereof, provided that R cannot denote 2-pyridinyl and cannot denote phenyl, 2,5-dichlorophenyl or 4-methylphenyl if X¹ and X² are at the same time hydrogen, or R cannot denote 3,4-dichloro-phenyl if X¹ or X² represent methyl.

2. 3-(4-Pyridinyl-imino)-3H-[1,2,4]-thiadiazolo [4,3-a]-pyridine as claimed in claim 1 as well as physiologically tolerated salts thereof.

3. Process for producing compounds as claimed in claim 1, wherein a compound of formula II 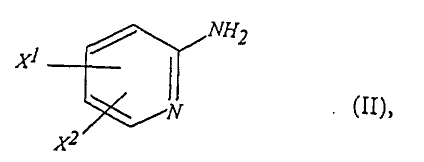 in which X¹ and X² have the meaning described above is reacted in a known manner either
a) with a compound of formula III
CISCCl₃ (III),
or a reactive derivative thereof
and a compound of the general formula IV
H₂N-R (IV),
in which R has the above-mentioned meaning or
b) with a compound of the general formula V
YS-CY=N-R (V),
in which R has the above-mentioned meaning and Y represents a halogen atom
and subsequently a residue R is converted if desired into another residue given by the definition and the compound obtained of formula I is converted into a salt by reaction with physiologically tolerated acids or bases.

4. Pharmaceutical preparation containing a compound of the general formula I as claimed in claim 1 in addition to common carrier and auxiliary substances.

5. Pharmaceutical preparation containing the compound of claim 2 in addition to common carrier and auxiliary substances.

6. Compounds of the general formula I as claimed in claim 1 to produce pharmaceutical preparations for the treatment of allergic diseases.

7. Compound of claim 2 to produce pharmaceutical preparations for the treatment of allergic diseases.

## Revendications

1. Composés de formule I 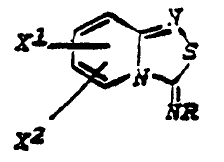 dans laquelle
X¹ et X² sont identiques ou différents et signifient un atome d'hydrogène, un résidu alkyle en C₁ à C₆ ou un atome d'halogène ou, pour autant qu'ils se trouvent dans des positions voisines, forment avec les atomes de carbone qui les portent, un cycle phényle condensé et
R signifie un résidu phényle, un résidu cyclohexyle, un résidu cyclopentyle, un résidu pyridyle, un résidu pipéridinyle, un résidu pyrimidinyle, un résidu pyrrolidinyle, un résidu isoxazolyle, un résidu oxazolyle, un résidu thiazolyle, un résidu thiazolinyle, un résidu thiazolyle ou un résidu tétrazolyle, qui peuvent si souhaité être monosubstitués ou polysubstitués par un atome d'halogène, un groupe cyano, un groupe nitro, un groupe alkyle en C₁ à C₆, un groupe halogéno(alkyle en C₁ à C₆), un groupe hydroxy, un groupe alcoxy en C₁ à C₆, un groupe méthylènedioxy, un groupe (alkyle en C₁ à C₆)thio, un groupe halogéno(alkyle en C₁ à C₆)thio, un groupe amino, un groupe (alkyle en C₁ à C₆)amino, un groupe (dialkyle en C₂ à C₁₂)amino, un groupe pyrrolyle, un groupe carboxy, un groupe carbamoyle, un groupe benzyle, un groupe hydroxy(alkyle en C₁ à C₆), un groupe carboxy(alkyle en C₂ à C₇), un groupe (alcoxy en C₂ à C₇)carbonyle-alkyle en C₁ à C₆, un groupe carbamoyle-alkyle en C₁ à C₆, un groupe N-hydroxy-N-(alkyle en C₁ à C₆)-carbamoyl-(alkyle en C₁ à C₆) ou un groupe alcényle en C₂ à C₆ ainsi que leurs sels physiologiquement acceptables, sous réserve que R ne puisse pas signifier un groupe 2-pyridinyle et ne puisse pas signifier un groupe phényle, ni un groupe 2,5-dichlorophényle, ni un groupe 4-méthylphényle lorsque X¹ et X² sont simultanément un atome d'hydrogène, ou que R ne puisse pas signifier un groupe 3,4-dichlorophényle lorsque X¹ ou X² représente un groupe méthyle.

2. 3-(4-pyridinyl-imino)-3H-[1,2,4]-thiadiazolo[4,3-a]-pyridine selon la revendication 1 ainsi que ses sels physiologiquement acceptables.

3. Procédé pour la préparation de composés selon la revendication 1, **caractérisé en ce qu'**on transforme de manière connue en soi un composé de formule II 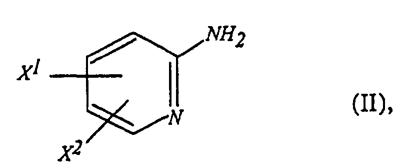 dans laquelle X¹ et X² ont la signification susmentionnée avec
a) un composé de formule III
ClSCCl₃ (III)
ou avec un dérivé réactif de celui-ci
et un composé de formule générale IV
H₂N-R (IV)
dans laquelle R a la signification susmentionnée ou
b) un composé de formule générale V
YS-CY=N-R
dans laquelle R a la signification susmentionnée et Y signifie un atome d'halogène
et **en ce qu'**on transforme ensuite, si souhaité, un radical R en un autre radical indiqué par la définition et **en ce qu'**on transforme le composé obtenu de formule I si souhaité par transformation avec des acides ou des bases physiologiquement acceptables en un sel.

4. Médicament contenant un composé de formule générale I selon la revendication 1, en plus de supports et d'adjuvants usuels.

5. Médicament contenant un composé de la revendication 2, en plus de supports et d'adjuvants usuels.

6. Composés de formule générale I selon la revendication 1 pour la préparation de médicaments pour le traitement de maladies allergiques.

7. Composé de la revendication 2 pour la préparation de médicaments pour le traitement de maladies allergiques.
